# EUROPÄISCHE PATENTSCHRIFT

(11) **EP 0 950 131 B1**
(45) Veröffentlichungstag und Bekanntmachung des Hinweises auf die Patenterteilung: **12.12.2001**
(21) Anmeldenummer: 97945783.5
(22) Anmeldetag: 23.10.1997
(51) Int. Cl.: C25D 11/02

(54) **METALLISCHER GEGENSTAND MIT EINER DÜNNEN MEHRPHASIGEN OXIDSCHICHT SOWIE VERFAHREN ZU DESSEN HERSTELLUNG**
METALLIC OBJECT WITH A THIN POLYPHASE OXIDE COATING AND PROCESS FOR THE MANUFACTURE THEREOF
OBJET METALLIQUE A FINE COUCHE D'OXYDE POLYPHASEE ET PROCEDE PERMETTANT DE LE PRODUIRE

(30) Priorität: 24.10.1996 DE 19643555
(43) Veröffentlichungstag der Anmeldung: 20.10.1999
(73) Patentinhaber: Merck Patent GmbH, 64293 Darmstadt (DE)
(72) Erfinder: WORCH, Hartmut, D-01069 Dresden (DE); THIEME, Michael, D-01326 Dresden (DE); SCHARNWEBER, Dieter, D-01324 Dresden (DE); RÖSSLER, Sophie, D-01159 Dresden (DE); STÖLZEL, Martina, D-01723 Kesselsdorf (DE)
(86) Internationale Anmeldenummer: DE9702465
(87) Internationale Veröffentlichungsnummer: WO9817844

(56) Entgegenhaltungen:
- EP-A- 0 232 791

## Beschreibung

Die Erfindung betrifft einen metallischen aus einem Ventilmetall oder dessen Legierung einschließlich intermetallische Phasen bestehender Gegenstand mit einer dünnen mehrphasigen Oxidschicht und ein Verfahren zu dessen Herstellung. Gegenstände mit einer derartigen Oxidschicht weisen neben vorteilhaften chemischen und physikalischen Eigenschaften eine hohe Biokompatibilität auf und sind aufgrund ihrer Eigenschaften vielfältig einsetzbar.

Bislang bekannte mehrphasige Oxidschichten auf metallischen Werkstoffen werden mit Verfahren erzeugt, die Interdiffusionsprozesse bei hohen Temperaturen ausnutzen oder über Auftragungsverfahren mit wechselnder Schichtfolge (Flammspritzen, PVD-Verfahren) Mehrphasigkeit zu erreichen suchen. Auch aus der Sol-Gel-Technologie sind über Behandlungen bei hohen Temparaturen entsprechende Schichten herstellbar.
Allen diesen Verfahren ist gemeinsam, daß sie zumindest teilweise bei Prozeßbedingungen durchgeführt werden, die insbesondere aufgrund hoher Temperaturen den Einbau organischer Phasen unmöglich machen sowie bei anorganischen Phasen überwiegend zum Einbau wasserfreier Hochtemperaturmodifikationen führen.

Ein Verfahren zur Erzeugung von gegebenenfalls modifizierten Oxidkeramikschichten auf sperrschichtbildenden Metallen (Ventilmetalle; Ti, Al, Zr etc.) wird in EP 0545230 vorgestellt. Diese Oxidkeramikschichten werden durch eine plasmachemische anodische Oxidation in einem chloridfreien Elektrolytbad mit einem pH-Wert von 2 bis 8 Reaktion bei Temperaturen von -30 bis +15 °C durchgeführt. Bei diesem Verfahren kommt es zu keiner Legierungsbildung zwischen der Metalloxidphase und weiteren anorganischen Phasen. Aufgrund der am Ort der Oxidbildung lokal vorliegenden plasmachemischen Bedingungen werden eventuell vorliegende organische Substanzen vollständig zerstört. Aus DE-OS 36 27 249 ist ein Verfahren bekannt, mit dessen Hilfe Konversionsschichten auf Titanoberflächen erzeugt werden, die aus hochmolekularen organischen Verbindungen und Tensiden bestehen. Diese Schichten sind durch eine sehr gute Haftfestigkeit gekennzeichnet, werden jedoch nicht über eine Legierungsbildung von Metalloxid und einer weiteren Phase realisiert. Die Beschichtung erfolgt außerdem bei Temperaturen von 40 - 80 °C, wodurch die Verwendung von Proteinen ausgeschlossen wird.

Aus EP 0232791 und EP 0237053 sind Verfahren bekannt, bei denen durch anodische Oxidation unter Funkenentladung in wäßrigen Elektrolyten eine in Oxiden enthaltene, resorbierbare Calciumphosphatkeramik auf Titan aufgebracht wird. Die dabei erzeugten Schichten bestehen aber nicht aus Hydroxylapatit oder Flourapatit, sondern aus Oxiden und stark resobierbaren Caliumphosphaten. Mit der vollständigen Resorption der Calciumphosphatphasen geht dabei auch der bioaktive Charakter des Implantats verloren. Da auch hier die Oxidschichtbildung unter Funkenentladung erfolgt, werden eventuell vorliegende organische Substanzen vollständig zerstört.

Das Verfahren nach EP 232 791 schließt aufgrund der bei Funkenentladung auftretenden Temperaturen von weit über 1000°C den Einbau empfindlicher Komponenten völlig aus.

In CA 2,073,781 A1 wird ein Verfahren vorgestellt, bei dem durch anodische Oxidation der eingesetzten Metalle (Titan) bzw. Legierungen (Ti- und Co-Basislegierungen) eine Oxidschicht gebildet und durch daran sich anschließender katodischer Polarisation Calciumphosphatphasen mit unterschiedlicher Kristallmodifikation auf der anodisch gebildeten Oxidschicht abgeschieden werden. Die auf diesem Wege erzeugten Schichten sollen mit biologisch aktiven Substanzen, wie zum Beispiel Kollagene, BMP (bone morphogenetic proteins) oder Antibiotika behandelt werden. Mit diesem Verfahren lassen sich die organischen Phasen nicht in die elektrochemisch gebildeten Oberflächenschichten einbauen.

Die WO 92/13984 beschreibt ein Verfahren zur Abscheidung bioaktiver Schichten auf leitfähigen Substraten. In einer Elektrolysezelle ist eine inerte Anode und eine Elektrolytlösung enthalten, die aus einer wäßrigen Lösung von Ionen der Keramik besteht und einen pH-Wert von weniger als 8 aufweist. Das aktivierte leitfähige Substrat wird in die Elektrolytlösung eingetaucht und das Potential zwischen Anode und leitfähigem Substrat so eingestellt, daß durch eine pH-Wert-Erhöhung an der Nahtstelle zwischen Elektrolytlösung und leitfähigem Substrat eine keramische Schicht auf dem leitfähigen Substrat abgeschieden wird. Nachteilig an der Lösung ist, daß die Schichtabscheidung nur auf der Oberfläche des Substrats erfolgt, so daß zum einen keine belastbare Verbindung zu dieser entstehen kann und die Schicht zum anderen biologisch vollständig resorbierbar ist.

Die Aufgabe der Erfindung besteht in der Schaffung metallischer Gegenstände mit verbesserter Oberflächenbeschichtung durch Erzeugung dünner mehrphasiger Oxidschichten unter Prozeßbedingungen, die den Einbau organischer und/oder anorganischer Phasen ermöglichen.

Erfindungsgemäß wird die Aufgabe durch einen metallischen aus einem Ventilmetall oder dessen Legierung einschließlich intermetallische Phasen bestehender Gegenstand mit einer dünnen mehrphasigen Oxidschicht aus einem metallischen Oxidphase und mindestens einer weiteren organischen und/oder anorganischen Komponente gelöst, erhältlich dadurch, dass die metallische Substratwekstoff so in Kontakt mit einer in die Oxidschicht zu integrienenden organischen und/oder anorganischen Phase gebracht wird, dass diese an der Substratoberfläche vorliegen und der so behandelte Substratwekstoff parallel dazu oder anschließend anodisch in einer Elektrolytlösung polarisiert wird.

Dabei wird der metallische Substratwerkstoff so in Kontakt mit einer in die Oxidschicht zu integrierenden organischen und/oder anorganischen Phase gebracht, dass diese an der Substratoberfläche vorliegen. Der so behandelte Substratwerkstoff wird parallel dazu oder anschließend anodisch in einer Elektrolytlösung polarisiert. Der metallische Gegenstand besteht aus einem Ventilmetall wie z.B. Aluminium, Titan, Tantal, Zirkonium, Niob oder dessen Legierung einschließlich intermetallischer Phasen. Auf solchen Metallen bzw. Legierungen ausgebildete Oxidschichten weisen zumindest bei anodischer Polarisation lonenleitung auf und erlauben damit über anodische Polarisation eine Variation der Oxidschichtdicke in weiten Grenzen.

Die Aufteilung des Oxidschichtwachstums auf die Phasengrenzen metallischer Substratwerkstoff/Oxid sowie Oxid/Umgebung kann dabei durch die Wahl der elektrochemischen Bedingungen kontrolliert werden. Auf diese Weise lassen sich zweischichtige Oxidschichten erzeugen, deren äußerer Schichtanteil anorganische und/oder organische Phasen enthalten kann, wobei über die Wahl der elektrochemischen Parameter Potential, Strom und Potentialänderungsgeschwindigkeit sowohl die Gesamtdicke der Oxidschicht als auch die Aufteilung der Gesamtdicke auf die beiden Schichtbestandteile kontrolliert werden kann. Dadurch wird es möglich, je nach der Teilchengröße der in die Oxidschicht zu integrierenden Phasen diese entweder vollständig zu integrieren bzw. einen definierten Integrationsgrad einzustellen.

Die organische Komponente besteht aus vorzugsweise aus Polymermaterialien wie z.B. Kollagen, S-Layer, Polykarbonat und Fullerenen und/oder aus Biomolekülen und/oder aus Oligomeren.
Die anorganische Komponente wird vorzugsweise von anorganischen Faserstrukturen oder Calciumphosphatphasen gebildet. Sie kann allein oder in Verbindung mit der organischen Komponente bzw. als Verbund mit der organischen Komponente in die Oxidphase des Metallwerkstoffes eingebaut sein.

Die organische und/oder anorganische Komponente ist erfindungsgemäß in die metallische Oxidphase so eingebaut, daß die mehrphasige Oxidschicht einer Legierung gleichkommt. Die organische Komponente kann über die mehrphasige Oxidschicht hinausreichen.

Erfindungsgemäß wird eine dünne mehrphasige Oxidschicht auf einem metallischem Substratwerkstoff so erzeugt, daß zunächst der metallischer Substratwerkstoff so in Kontakt mit den in die Oxidschicht zu integrierenden organischen und/oder anorganischen Phasen gebracht wird, daß diese an der Substratoberfläche vorliegen.
Der Kontakt mit den in die Oxidschicht zu integrierenden Phasen kann durch Adsorption, Sedimentation, Auftragung, Abscheidung bzw. innigen mechanischen Kontakt oder Die organische und/oder anorganische Komponente ist erfindungsgemäß in die metallische Oxidphase so eingebaut, daß die mehrphasige Oxidschicht einer Legierung gleichkommt. Die organische Komponente kann über die mehrphasige Oxidschicht hinausreichen.

Erfindungsgemäß wird eine dünne mehrphasige Oxidschicht auf einem metallischem Substratwerkstoff so erzeugt, daß zunächst der metallischer Substratwerkstoff so in Kontakt mit den in die Oxidschicht zu integrierenden organischen und/oder anorganischen Phasen gebracht wird, daß diese an bzw. in unmittelbarer Nähe der Substratoberfläche vorliegen.
Der Kontakt mit den in die Oxidschicht zu integrierenden Phasen kann durch Adsorption, Sedimentation, Auftragung, Abscheidung bzw. innigen mechanischen Kontakt oder durch Einbringung in bzw. Aufbringung von Suspensionen der zu integrierenden Phasen realisiert werden. Der Transport der in die Oxidschicht zu intergrierenden Phasen zur Substratoberfläche kann dabei durch Anwendung elektromagnetischer Felder durchgeführt bzw. unterstützt werden.
Parallel dazu oder anschließend wird in einem elektrochemischen Verfahrensschritt der die Substratoberfläche darstellende Werkstoff in einer Elektrolytlösung anodisch polarisiert.
Dieser Verfahrensschritt führt bei aus Ventilmetallen oder deren Legierungen bestehenden metallischen Werkstoffen zu einem Oxidschichtwachstum an der Phasengrenze Oxidschicht/Umgebung über Lösungs-Fällungs-Reaktionen, in dessen Folge die an der bzw. in unmittelbarer Nähe dieser Phasengrenze vorliegenden Phasen vollständig oder partiell in die neugebildete Oxidschicht integriert werden.

Die vorgenannten Verfahrensschritte werden für den Fall des Einbaus physiologischer, organischer Komponenten bei oder nahe Raumtemperatur durchgeführt, so daß sowohl Struktur als auch Funktion dieser Komponenten erhalten bleiben.

Die anodische Polarisation kann galvanostatisch, potentiostatisch oder potentiodynamisch bis zum Erreichen eines vorbestimmten Formierungspotentials geführt werden. Kriterium für die Auswahl der Bedingungen bei der anodischen Polarisation ist, daß im Zuge der Bildung der *dünnen* mehrphasigen Oxidschichten Struktur und Funktion der einzuschließenden Komponenten optimal erhalten bleiben.

Das Formierungspotential liegt zwischen 2 und 200 V_{SCE}.

Der Vorteil der erfindungsgemäß erzeugten Schichten besteht darin, daß durch den festen Einbau der organischen und/oder organischen Komponente in die Oxidschicht des Metallwerkstoffes eine verbesserte Kraftübertragung und eine dauerhafte Verbesserung der Biokompatibilität erreicht werden.

### Anhand nachfolgender Ausführungsbeispiele wird die Erfindung näher erläutert:

### Ausführungsbeispiel 1

Aus säurelöslichem gefriergetrockneten Kalbshautkollagen wird eine Kollagenlösung hergestellt. Dazu wird das Kollagen vom Typ I in 0,01 M Essigsäure gelöst und anschließend auf eine Konzentration von 0,36 mg/ml bei 4 °C und pH = 3,5 eingestellt. Die Rekonstitution der Kollagenmoleküle erfolgt in zwei Prozeßschritten; der pH-Wert-Einstellung auf 7,4 in doppelt konzentriertem Phosphatpuffer und der Temperaturerhöhung auf 34 °C. Nach 3 Stunden besteht die Lösung aus nativ rekonstituierten Kollagen I-Fibrillen.

Eine zylindrische Probe aus Ti6A14V mit einem Durchmesser von 9 mm, einer Dicke von 6 mm wird geschliffen (25 - 7 µm) und oxidpoliert. Anschließend wird die Probe in Alkohol gereinigt und mit deionisiertem Wasser gespült. Die so vorbehandelte Probe wird in die Kollagenlösung vertikal eingebracht, so daß die polierte Probenoberfläche vollständig bedeckt ist. Auf der Probenoberfläche wird dann natives Kollagen I adsorbiert. Die Adsorptionsdauer beträgt 20 Minuten.

Nach Adsorption wird die Metallprobe aus der Kollagenlösung herausgenommen, mit destilliertem Wasser gespült und als Substratelektrode in eine Drei-Elektroden-Anordnung aus einer gesättigten Kalomelelektrode als Bezugselektrode und einem Platinblech als Gegenelektrode in einer thermostatierten Elektrolysezelle eingebracht. Als Elektrolytlösung dient eine schwach basische Phosphatlösung. Die elektrochemische Reaktion wird bei 34°C in einer Doppelmantelzelle durchgeführt. Die Substratelektrode wird in dieser Anordnung mit einer Potentialänderungsgeschwindigkeit von 2 V/sec potentiodynamisch bis zu einem Formierungspotential von 100 V anodisch polarisiert. Die Probe wird aus dem Elektrolysebad herausgenommen, mit deionisiertem Wasser gespült und luftgetrocknet.

Elektronenmikroskopische Untersuchungen zeigen native Kollagen I-Fibrillen, die z.T. vollständig, z.T. partiell in die während der anodischen Polarisation gebildete Oxidschicht eingebaut wurden. Eine Querschnittspräparation der Titanoxidschicht weist eine Oxidschichtdicke von ca. 250 nm auf und zeigt die Abdrücke der eingebauten Fibrillen, deren Durchmesser denen der adsorbierten Fibrillen entsprechen.

### Ausführungsbeispiel 2

Aus säurelöslichem gefriergetrockneten Kalbshautkollagen wird eine Kollagenlösung hergestellt. Dazu wird das Kollagen vom Typ I in 0,01 M Essigsäure gelöst und anschließend auf eine Konzentration von 1 mg/ml bei 4 °C und pH = 3,5 eingestellt. Die Rekonstitution der Kollagenmoleküle erfolgt in zwei Prozeßschritten; der pH-Wert-Einstellung auf 7,4 in doppelt konzentriertem Phosphatpuffer und der Temperaturerhöhung auf 34 °C. Nach 3 Stunden besteht die Lösung aus nativ rekonstituierten Kollagen I-Fibrillen.

Eine zylindrische Probe aus Aluminium mit einem Durchmesser von 9 mm, einer Dicke von 6 mm wird geschliffen (25 - 7 µm) und oxidpoliert. Anschließend wird die Probe in Alkohol gereinigt und mit deionisiertem Wasser gespült. Auf die so vorbehandelte Probe wird Kollagenlösung aufgetropft, so daß die polierte Probenoberfläche vollständig bedeckt ist. Auf der Probenoberfläche wird dann natives Kollagen I adsorbiert. Die Adsorptionsdauer beträgt 40 Minuten.
Nach Adsorption wird die Kollagenlösung abgespült, mit destilliertem Wasser gespült und die Aluminiumprobe als Substratelektrode in eine Drei-Elektroden-Anordnung aus einer gesättigten Kalomelelektrode als Bezugselektrode und einem Platinblech als Gegenelektrode in einer thermostatierten Elektrolysezelle eingebracht. Als Elektrolytlösung dient doppelt konzentrierter Phosphat-Puffer pH = 7,4. Die elektrochemische Reaktion wird bei 34°C in einer Doppelmantelzelle durchgeführt. Die Substratelektrode wird in dieser Anordnung mit einer Stromdichte von 3 mA/cm² bis zu einem Formierungspotential von 40 V anodisch polarisiert. Unmittelbar folgend wird dieses Potential für 100 s potentiostatisch fixiert. Nach Abschalten der Polarisation wird die Probe wird aus dem Elektrolysebad herausgenommen, mit deionisiertem Wasser gespült und luftgetrocknet.

Elektronenmikroskopische Untersuchungen zeigen native Kollagen I-Fibrillen, die an den Stellen, an denen sie auf der Luftoxidschicht des Aluminiums auflagen, partiell in die während der anodischen Polarisation gebildete Oxidschicht eingebaut sind.

### Ausführungsbeispiel 3

Eine zylindrische Probe aus der Legierung Ti-6A1-4V mit einem Durchmesser von 9 mm, einer Dicke von 6 mm wird geschliffen (25 - 7 µm) und oxidpoliert. Anschließend wird die Probe in Alkohol gereinigt und mit deionisiertem Wasser gespült. In einer Vakuumapparatur wird die Oberfläche der Probe mit C₆₀-Molekülen bedampft.

Nachfolgend wird die Probe als Substratelektrode in eine Drei-Elektroden-Anordnung aus einer gesättigten Kalomelelektrode als Bezugselektrode und einem Platinblech als Gegenelektrode in einer thermostatierten Elektrolysezelle eingebracht. Als Elektrolytlösung dient doppelt konzentrierter Phosphat-Puffer pH = 7,4. Die elektrochemische Reaktion wird bei 34°C in einer Doppelmantelzelle durchgeführt. Die Substratelektrode wird in dieser Anordnung mit einer Stromdichte von 0,5 mA/cm² für 500 s anodisch polarisiert. Dabei werden Formierungspotentiale bis etwa 8 V gemessen. Nach Abschalten der Polarisation wird die Probe wird aus dem Elektrolysebad herausgenommen, mit deionisiertem Wasser gespült und luftgetrocknet.
Elektronenmikroskopische Untersuchungen zeigen eine typische Titanoxid-Oberfläche. FT-IR-Untersuchungen im Reflexionsmode weisen in dieser Oberfläche das Vorhandensein von C₆₀-Molekülen nach.

## Patentansprüche

1. Metallischer, aus einem Ventilmetall oder dessen Legierung einschließlich intermetallischer Phasen bestehender Gegenstand mit einer dünnen mehrphasigen Oxidschicht aus einer metallischen Oxidphase und mindestens einer weiteren organischen und/oder anorganischen Komponente, erhältlich dadurch, dass der metallische Substratwerkstoff so in Kontakt mit einer in die Oxidschicht zu integrierenden organischen und/oder anorganischen Phase gebracht wird, dass diese an der Substratoberfläche vorliegen und der so behandelte Substratwerkstoff parallel dazu oder anschließend anodisch in einer Elektrolytlösung polarisiert wird.

2. Gegenstand nach Anpsruch 1, **dadurch gekennzeichnet, daß** der metallische Gegenstand aus Aluminium, Titan, Tantal, Zirkonium, Niob oder deren Legierungen einschließlich intermetallischer Phasen besteht.

3. Gegenstand nach Anspruch 1, **dadurch gekennzeichnet, daß** die organische Komponente insbesondere aus Polymermaterialien und/oder Biomolekülen und/oder Oligomeren besteht.

4. Gegenstand nach Anspruch 1 und 3, **dadurch gekennzeichnet, daß** die organische Komponente vorzugsweise aus Kollagen, S-Layer, Polykarbonat oder Fullerenen besteht.

5. Gegenstand nach Anspruch 1, **dadurch gekennzeichnet, daß** die anorganische Komponente vorzugsweise aus anorganischen Fasersturkturen oder Calciumphosphatphasen besteht.

6. Gegenstand nach einem der Ansprüche 1 bis 5, **dadurch gekennzeichnet, daß** die anorganische Komponente allein oder in Verbindung mit der organischen Komponente bzw. als Verbund mit der organischen Komponente in die Oxidschicht integriert ist.

7. Gegenstand nach einem der Ansprüche 1 bis 6, **dadurch gekennzeichnet, daß** die organische und/oder anorganische Komponente vollständig in die Oxidschicht integriert ist.

8. Gegenstand nach einem der Ansprüche 1 bis 6, **dadurch gekennzeichnet, daß** die organische und/oder anorganische Komponente in die Oxidschicht integriert ist und über diese hinausreicht.

9. Verfahren zur Herstellung einer dünnen mehrphasigen Oxidschicht auf metallischen Substratwerkstoffen, **dadurch gekennzeichnet, dass** der metallische Substratwerkstoff so in Kontakt mit einer in die Oxidschicht zu integrierenden organischen und/oder anorganischen Phase gebracht wird, dass diese an der Substratoberfläche vorliegen und dass der so behandelte Substratwerkstoff parallel dazu oder anschließend anodisch in einer Elektrolytlösung polarisiert wird.

10. Verfahren nach Anspruch 9, **dadurch gekennzeichnet, daß** der Kontakt zwischen Oxidschicht und zu integrierenden Phasen durch Adsorption, Sedimentation, Auftragung, Abscheidung bzw. innigen mechanischen Kontakt oder durch Einbringung in bzw. Aufbringung von Suspensionen der zu integrierenden Phasen realisiert wird.

11. Verfahren nach Anspruch 9, **dadurch gekennzeichnet, daß** der Transport der in die Oxidschicht zu integrierenden Phasen zur Substratoberfläche durch Anwendung elektromagnetischer Felder durchgeführt bzw. unterstützt wird.

12. Verfahren nach Anspruch 9, **dadurch gekennzeichnet, daß** die anodische Polarisierung bis zu einem Formierungspotential zwischen 2 und 200 V_{SCE} erfolgt.

13. Verfahren nach Anspruch 9 und 12, dadurch gekennnzeichnet, daß die anodische Polarisation bis zum Formierungspotential galvanostatisch, potentiostatisch oder potentiodynamisch ausgeführt wird.

## Claims

1. Metallic article consisting of a valve metal or its alloy including intermetallic phases and having a thin, multiphase oxide layer comprising a metallic oxide phase and at least one further organic and/or inorganic component, which can be obtained by the metallic substrate material being brought into contact in such a way with an organic and/or inorganic phase to be integrated into the oxide layer, that these are present at the substrate surface and the substrate material thus treated is polarized anodically in an electrolyte solution, either in parallel or subsequently.

2. Article according to Claim 1, **characterized in that** the metallic article consists of aluminium, titanium, tantalum, zirconium, niobium or alloys thereof, including intermetallic phases.

3. Article according to Claim 1, **characterized in that** the organic component consists, in particular, of polymer materials and/or biomolecules and/or oligomers.

4. Article according to Claim 1 and 3, **characterized in that** the organic component preferably consists of collagen, S-layer, polycarbonate or fullerenes.

5. Article according to Claim 1, **characterized in that** the inorganic component preferably consists of inorganic fibre structures or calcium phosphate phases.

6. Article according to any one of Claims 1 to 5, **characterized in that** the inorganic component, either on its own or in combination with the organic component or as a composite with the organic component, is integrated into the oxide layer.

7. Article according to any one of Claims 1 to 6, **characterized in that** the organic and/or inorganic component is completely integrated into the oxide layer.

8. Article according to any one of Claims 1 to 6, **characterized in that** the organic and/or inorganic component is integrated into the oxide layer and extends beyond it.

9. Method of producing a thin multiphase oxide layer on metallic substrate materials, **characterized in that** the metallic substrate material is brought into contact in such a way with an organic and/or inorganic phase to be integrated into the oxide layer, that these are present at the substrate surface and the substrate material thus treated is polarized anodically in an electrolyte solution, either in parallel or subsequently.

10. Method according to Claim 9, **characterized in that** the contact between oxide layer and phases to be integrated is effected by adsorption, sedimentation, application, deposition or internal mechanical contact, or by incorporating into, or application of, suspensions of the phases to be integrated.

11. Method according to Claim 9, **characterized in that** the transport of the phases to be integrated into the oxide layer to the substrate surface is effected or assisted or by applying electromagnetic fields.

12. Method according to Claim 9, **characterized in that** the anodic polarization takes place up to a forming potential of between 2 and 200 V_{SCE}.

13. Method according to Claim 9 and 12, **characterized in that** the anodic polarization up to the forming potential is effected galvanostatically, potentiostatically or potentiodynamically.

## Revendications

1. Objet métallique composé d'un métal pour soupapes ou d'un alliage de celui-ci, y compris des phases intermétalliques, avec une fine couche d'oxyde polyphasique faite d'une phase oxyde métallique et d'au moins un autre composant organique et/ou inorganique, pouvant être obtenu en mettant en contact le matériau de substrat métallique avec une phase organique et/ou inorganique à intégrer dans la couche d'oxyde de façon que ladite phase soit présente sur la surface du substrat et en polarisant anodiquement en parallèle ou par la suite le matériau de substrat ainsi traité dans une solution d'électrolyte.

2. Objet selon la revendication 1, **caractérisé en ce que** l'objet métallique se compose d'aluminium, de titane, de tantale, de zirconium, de niobium ou d'alliages de ceux-ci, y compris des phases intermétalliques.

3. Objet selon la revendication 1, **caractérisé en ce que** le composant organique se compose notamment de matériaux polymères et/ou de biomolécules et/ou d'oligomères.

4. Objet selon la revendication 1 et la revendication 3, **caractérisé en ce que** le composant organique se compose de préférence de collagène, de couche S, de polycarbonate ou de fullerènes.

5. Objet selon la revendication 1, **caractérisé en ce que** le composant inorganique se compose de préférence de structures fibreuses inorganiques ou de phases phosphate de calcium.

6. Objet selon l'une des revendications 1 à 5, **caractérisé en ce que** le composant inorganique est intégré dans la couche d'oxyde soit seul, soit combiné au composant organique, soit sous la forme d'un composite avec le composant organique.

7. Objet selon l'une des revendications 1 à 6, **caractérisé en ce que** le composant organique et/ou inorganique est complètement intégré dans la couche d'oxyde.

8. Objet selon l'une des revendications 1 à 6, **caractérisé en ce que** le composant organique et/ou inorganique est intégré dans la couche d'oxyde et se trouve en excès par rapport à celle-ci.

9. Procédé de production d'une fine couche d'oxyde polyphasique sur des matériaux substrats métalliques, **caractérisé en ce que** le matériau substrat métallique est mis en contact avec une phase organique et/ou inorganique à intégrer dans la couche d'oxyde de façon que ladite phase soit présente sur la surface du substrat et **en ce que** le matériau de substrat ainsi traité est polarisé anodiquement, en parallèle ou par la suite, dans une solution d'électrolyte.

10. Procédé selon la revendication 9, **caractérisé en ce que** le contact entre la couche d'oxyde et les phases à intégrer se fait par adsorption, sédimentation, recharge, dépôt ou contact mécanique intime ou bien par immersion dans ou revêtement avec des suspensions des phases à intégrer.

11. Procédé selon la revendication 9, **caractérisé en ce que** le transport des phases à intégrer dans la couche d'oxyde vers la surface du substrat est réalisé ou assisté au moyen de champs électromagnétiques.

12. Procédé selon la revendication 9, **caractérisé en ce que** la polarisation anodique se fait jusqu'à un potentiel d'activation allant de 2 à 200 V_{SCE}.

13. Procédé selon la revendication 9 et la revendication 12, **caractérisé en ce que** la polarisation anodique se fait jusqu'au potentiel d'activation par voie galvanostatique, potentiostatique ou potentiodynamique.
